# EUROPEAN PATENT SPECIFICATION

(11) **EP 2 793 856 B1**
(45) Date of publication and mention of the grant of the patent: **16.12.2015**
(21) Application number: 12834604.6
(22) Date of filing: 20.12.2012
(51) Int. Cl.: A61K 9/00, A61K 31/00, A61K 47/00

(54) **ORALLY-DISINTEGRATING FORMULATIONS OF FLURBIPROFEN**
IM MUND ZERFALLENDE FORMULIERUNGEN AUS FLURBIPROFEN
FORMULATIONS DE FLURBIPROFÈNE À DÉSINTÉGRATION ORALE

(30) Priority: 23.12.2011 TR 201112836; 27.12.2011 TR 201113006; 20.01.2012 TR 201200739
(43) Date of publication of application: 29.10.2014
(73) Proprietor: Sanovel Ilac Sanayi Ve Ticaret Anonim Sirketi, 34460 Istanbul (TR)
(72) Inventor: CIFTER, Umit, 34460 Istanbul (TR); TURKYILMAZ, Ali, 34460 Istanbul (TR); PEHLIVAN AKALIN, Nur, 34460 Istanbul (TR); ONDER, Ramazan, 34460 Istanbul (TR); SUCUOGLU, Esra, 34460 Istanbul (TR)
(74) Representative: Sevinç, Erkan
(86) International application number: PCT/TR2012/000243
(87) International publication number: WO 2013/095318

(56) References cited:
- EP-A2- 0 371 431
- GB-A- 2 432 526
- US-A- 4 996 209
- US-A1- 2004 039 366
- KENJIROU HIGASHI: "Simultaneous dissolution of naproxen and flurbiprofen from a novel ternary gamma-cyclodextrin complex", CHEMICAL AND PHARMACEUTICAL BULLETIN, PHARMACEUTICAL SOCIETY OF JAPAN, JP, vol. 58, no. 5, 1 May 2010 (2010-05-01), pages 769-772, XP009168502, ISSN: 0009-2363

## Description

### Field of Invention

The present invention relates to a formulation comprising flurbiprofen or a pharmaceutically acceptable salt thereof. The present invention more particularly relates to an orally-disintegrating tablet formulation of flurbiprofen, which is stable against environmental influences.

### Background of Invention

Flurbiprofen is a propionic acid derivative, also known as NSAID (non-steroidal anti-inflammatory drug), having analgesic and anti-inflammatory activities. Its chemical structure is illustrated with Formula 1 given below.

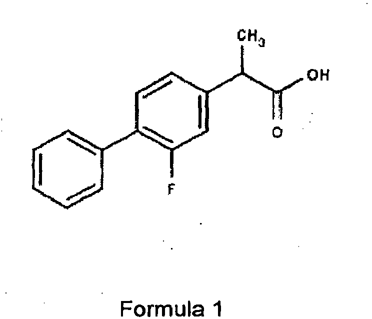

Flurbiprofen is used for alleviating pain in muscle-skeleton system and joint disorders such as ankylosing spondylitis, osteoarthritis, and rheumatoid arthritis, in soft tissue injuries such as sprains and strains, in postoperative cases, and in painful and severe menstruation and migraine. Flurbiprofen is further used as a lozenge in the symptomatic amelioration of sore throats.

Flurbiprofen sodium is used in eye drops for preventing intraoperative miosis, as well as for controlling the postoperative inflammation of the anterior layer of the eye. Flurbiprofen axetil is administered via intravenous injection against severe pains in some countries.

The application WO 98/52545 relates to pharmaceutical compositions comprising a formulation of flurbiprofen with a therapeutically effective amount of one or more active ingredients selected from an antihistamine, a cough suppressant, a decongestant, an expectorant, a muscle relaxant, a centrally acting analgesic, a local anesthetic, an antibacterial compound, an antiviral compound, an antibiotic compound, an antifungal compound, minerals and vitamins and/or a burn-masking amount of an agent which has a warming effect on the mucosa of the throat.

The patent US05807568 discloses a topically-administered formulation comprising flurbiprofen as the active agent.

The patent WO9523596 discloses a flurbiprofen solution in a C2-4 alcohol.

The use of flurbiprofen in treating local pains and inflammations may cause a problem especially for those who have gastrointestinal system disorders. Flurbiprofen leads to a complaint in the form of burning sensation in the gastrointestinal system. Preventing the systemic side effects of flurbiprofen is quite important in terms of patient compliance. Various coating processes have been performed to prevent such side effects. The coatings, however, may cause problems in terms of solubility and thus bioavailability. Enhancing the absorption rate both provides ease of application and increases the molecule's efficiency.

Another problem in relation to flurbiprofen is that this molecule is poorly soluble in water. This, in turn, directly effects the bioavailability, and therefore the solubility and dissolution rate have to be increased.

Flurbiprofen is presented in many conventional tablet formulations. This active pharmaceutical agent used for treating many diseases, however, is preferred in an orally-disintegrating form. These preparations are advantageous for use in children, elder, and noncompliant patients. Orally-disintegrating dosage forms are convenient when no drinking water is available or is not preferable.

On the other hand, various difficulties are encountered in formulating the orally-disintegrating dosage forms. Developing orally-disintegrating formulations is difficult due to several different causes. First of all, the time required for the disintegration of the dosage form in the oral cavity under the presence of saliva is much shorter than the time required by the stomach. Therefore, these formulations are quite porous and therefore are not too hard. Such porous formulations are prone to be susceptible to humidity. As a result of this, some stability problems are encountered. In these formulations, the tablet hardness must be selected in a range that would ensure the balance between the disintegration time and the stability of the respective tablet. In addition to these, measures must be taken while preparing, packaging, handling, and storing finished dosage forms of orally-disintegrating formulations.

For this reason, orally-disintegrating compositions of flurbiprofen or a pharmaceutically acceptable salt thereof is desired, which would overcome the drawbacks referred to above and would let the active agent be disintegrated and dissolved in the oral cavity.

Apart from the stability problems possibly to arise from the porous structure of the orally-disintegrating tablet formulation of flurbiprofen, other stability problems are frequently encountered in formulations developed with flurbiprofen under the influence of environmental and physical conditions. Flurbiprofen is an active agent that is highly-susceptible to air and humidity conditions. When flurbiprofen is initially exposed to air and humidity, it degrades structurally and develops chemical behavioral changes. As a result of this, two main problems emerge. The first problem is that the stability of the products developed is not at a desired level and the shelf life thereof is shortened. Secondly, flurbiprofen may react with the excipients employed in developing those formulations containing the same. This, in turn, causes impurities and unwanted components to be included into the formulation.

In result, a novelty is required in the art of formulations having anti-inflammatory, analgesic, and antipyretic activities due to the aforesaid drawbacks.

### Object and Brief Description of Invention

The present invention provides an easily-administrable flurbiprofen formulation, eliminating all problems referred to above and bringing additional advantages to the relevant prior art.

Accordingly, the main object of the present invention is to obtain at least one stable formulation with anti-inflammatory, analgesic, and antipyretic activities.

Another object of the present invention is to provide a formulation having a desired solubility and dissolution rate, and therefore a desired level of bioavailability, with this formulation comprising flurbiprofen produced by means of a hot-melt method.

A further object of the present invention is to eliminate the need for any liquid solvent, including water.

Another object of the present invention is to obtain a uniform formulation content.

A further object of the present invention is to develop a formulation not leading to flowability problems during production.

Another object of the present invention is to provide a high disintegration rate for said formulation.

An orally-disintegrating pharmaceutical formulation has been developed to carry out any objects, referred to above and to emerge from the following detailed description.

According to a preferred embodiment of the present invention, said novelty is realized using flurbiprofen and polyvinylcaprolactam-polyvinyl acetate-polyethylene glycol graft copolymer.

According to another preferred embodiment of the present invention, said formulation is obtained by means of a hot-melt method not involving any liquid solvent during the granulation phase.

According to a preferred embodiment of the present invention, the proportion of flurbiprofen to polyvinylcaprolactam-polyvinyl acetate-polyethylene glycol graft copolymer is in the range of 0.1 to 10, preferably 0.15 to 5, and more preferably 0.2 to 2.

According to another preferred embodiment of the present invention, the hardness of the tablet is 5 to 100 N, preferably 10 to 60 N, and more preferably 15 to 40 N.

A further preferred embodiment of the present invention comprises at least one or more excipient(s).

According to another preferred embodiment of the present invention, one or more pharmaceutically acceptable excipients are selected from the group consisting of disintegrants, diluents, plasticizers, binders, glidants, lubricants, sweeteners, flavoring agents, and coloring agents.

According to another preferred embodiment of the present invention, said disintegrants are selected from the group consisting of at least one or a mixture of croscarmellose sodium, crospovidone, low-substituted hydroxypropyl cellulose, sodium starch glycolate, and soy polysaccharides.

According to another preferred embodiment of the present invention, the proportion by weight of flurbiprofen to the disintegrant is between 1:10 and 10:1.

According to another preferred embodiment of the present invention, said disintegrant is preferably croscarmellose sodium.

According to another preferred embodiment of the present invention, said diluent is preferably mannitol and/or microcrystalline cellulose.

According to another preferred embodiment of the present invention, the formulation disintegrates in the oral cavity in 90 seconds and preferably in 40 seconds.

According to another preferred embodiment of the present invention, said plasticizer comprises preferably at least one or a properly-proportioned mixture of castor oil, glycerin, citrate esters (acetyl tri-n-butyl citrate, acetyl triethyl citrate, tri-n-butyl citrate, triethyl citrate), sebacate esters (dibutyl sebacate), phthalate esters (diethyl phthalate, dibutyl phthalate, dioctyl phthalate), mineral oils, polyethylene oxide, triacetine, diethyl phthalate, fatty acid esters (butyl stearate, glycerol monostearate, stearyl alcohol), low-molecular weight glycol derivatives (polyethylene glycol, propylene glycol).

According to another preferred embodiment of the present invention, said binder is preferably polyvinylpyrrolidone and/or hydroxypropyl methyl cellulose.

According to another preferred embodiment of the present invention, said lubricant is preferably magnesium stearate and sodium stearyl fumarate.

According to another preferred embodiment of the present invention, said glidant is preferably colloidal silicon dioxide.

According to another preferred embodiment of the present invention, said sweetener is preferably aspartam, sucralose and/or saccharine.

According to another preferred embodiment of the present invention, said flavoring agent is preferably menthol and/or fruit extracts.

A further preferred embodiment of the present invention consist of,
(a) approximately 5 to 60% by weight of flurbiprofen or a pharmaceutically acceptable salt thereof;
(b) approximately 0.25 to 20% by weight of polyvinylcaprolactam-polyvinyl acetate-polyethylene glycol graft copolymer,
(c) approximately 0.1 to 30% by weight of croscarmellose sodium,
(d) approximately 1 to 60% by weight of mannitol,
(e) approximately 1 to 30% by weight of microcrystalline cellulose,
(f) approximately 1 to 20% by weight of polyvinylpyrrolidone,
(g) approximately 0.01 to 5% by weight of colloidal silicon dioxide,
(h) approximately 0.1 to 5% by weight of magnesium stearate,
(i) approximately 0.01 to 5% by weight of sucralose,
(j) approximately 0.01 to 5% by weight of menthol and/or fruit extracts.

Another preferred embodiment of the present invention provides a process for preparing an orally-disintegrating pharmaceutical formulation, this method comprising the steps of
(a) mixing flurbiprofen, plasticizer and polyvinylcaprolactam-polyvinyl acetate-polyethylene glycol graft copolymer together, melting this mixture, and passing it through an extruder or sieve,
(b) mixing the granules obtained above with other excipients;
(c) blending this mixture with a lubricant and a glidant;
(d) compressing the blended mixture into tablets.

### Detailed Description of Invention

The present invention is described with the following example in more details. This example is not limiting the scope of the present invention and must be considered under the light of the foregoing detailed disclosure. It is obvious that those skilled in the relevant art can produce similar embodiments under the light of the foregoing disclosures by making many adaptations on both the materials and methods, without departing from the scope of the present invention.

This orally-disintegrating tablet composition composed of the followings is designed to minimize the disintegration times, to enhance the solubility and dissolution rate, and maximize the mechanical resistance of the tablet according to the present invention.

### Example

An orally-disintegrating tablet composition is composed of the followings:
(a) approximately 5 to 60% by weight of flurbiprofen or a pharmaceutically acceptable salt thereof;
(b) approximately 0.25 to 20% by weight of polyvinylcaprolactam-polyvinyl acetate-polyethylene glycol graft copolymer,
(c) approximately 0.1 to 30% by weight of croscarmellose sodium,
(d) approximately 1 to 60% by weight of mannitol,
(e) approximately 1 to 30% by weight of microcrystalline cellulose,
(f) approximately 1 to 20% by weight of polyvinylpyrrolidone,
(g) approximately 0.01 to 5% by weight of colloidal silicon dioxide,
(h) approximately 0.1 to 5% by weight of magnesium stearate and sodium lauryl sulfate,
(i) approximately 0.01 to 5% by weight of sucralose,
(j) approximately 0.01 to 5% by weight of menthol and/or orange aroma.

This formulation is produced as follows. Flurbiprofen, plasticizer and polyvinylcaprolactam-polyvinyl acetate-polyethylene glycol graft copolymer are mixed together, this mixture is melted and passed through an extruder or sieve. The granules obtained preferably are admixed with ingredients, the resulting mixture is blended with lubricant(s) and glidant(s), and the blended mixture is compressed into tablets.

The orally-disintegrating formulations according to the present invention can be prepared by techniques such as direct compression, dry granulation, wet granulation, etc. well known to those skilled in the art. Orally-disintegrating compositions according to the present invention can also be prepared by means of technologies such as spray drying, freeze drying, floss formation process, die casting, Zydis^{®} technology, Flashtab technology, OraSolv^{®} technology, DuraSolv^{®} technology, Wowtab™ (nonaqueous immediately-soluble tablet) technology and other similar technologies. Flurbiprofen or a pharmaceutically acceptable salt thereof and other excipients are preferably mixed together and then the resulting mixture is compressed to give tablets.

With this invention, a stable formulation having a high solubility and a high dissolution rate is surprisingly obtained. Said formulation comprises flurbiprofen and polyvinylcaprolactam-polyvinyl acetate-polyethylene glycol greft copolymer or additionally at least one or a properly-proportioned mixture of polyvinyl alcohol-polyethylene glycol copolymer, polyoxyethylene-polyoxypropylene block copolymer, stearyl macrogol glyceride, polyethylene glycol, povidone, cationic methacrylate, copovidone, methacrylic acid copolymer derivatives, cellulose acetate phthalate, acetylated monoglyceride, dibutyl tartrate, diethyl phthalate, dimethyl phthalate, glycerin, propylene glycol, stearic acid, triacetine, triacetine citrate and tripropionin. The method described above both serves to provide a uniform formulation content, and eliminates the need for any liquid solvent including water. Any flowability problems encountered during production are prevented as well. In said formulation, the proportion of flurbiprofen to polyvinylcaprolactam-polyvinyl acetate-polyethylene glycol graft copolymer is in the range of 0.1 to 10, preferably 0.15 to 5, and more preferably 0.2 to 2. These ranges allow to achieve the desired dissolution rate and solubility. Polymers with low glass transition temperature and melting point are used in said formulation. On the other hand, using a plasticizer which reduces the glass transition temperature increases the stability of the active agent. The plasticizer used in the hot-melt method drops down the glass transition temperature of the polymers used in hot-melting, and thus allows to formulate the active agent at lower temperatures. In result, the formulation is made more stable. The formulation disintegrates in the oral cavity in 90 seconds and preferably in 40 seconds. The hardness of the tablet according to the present invention is 5 to 100 N, preferably 10 to 60 N, and more preferably 15 to 40 N. Disintegration is achieved in a desired time interval by applying a compressive force in said ranges and adding proper disintegrants. The preferred tablet hardness is in a range that would ensure the balance between the tablet's disintegration time and the stability. One of the important advantages of the developed formulation is the prevention or minimization of the burning sensation occurring in the gastrointestinal system by means of the matrix formed.

The present invention is used for the prevention or treatment of pain, arthralgia, toothache, myalgia, miosis inhibition, ankylosing spondylitis, osteoarthritis, rheumatoid arthritis and other muscle-skeleton system and joint disorders, soft tissue injuries such as sprains and strains, postoperative pains, painful and severe menstruation, migraine, and sore throat.

Orally-disintegrating formulations according to the present invention comprises tablets, single-dose packages, mini tablets, and multilayered and multicoated tablets, pellets, or powders formulated according to the standard methods of the relevant art. The formulation is preferably in the form of tablets.

The orally-disintegrating tablet composition according to the present invention is preferably in the form of a disk, circle, sphere, donut, bar, polygon, ellipse, etc..

The term granule, as used herein, means a powder, particle, or a pellet form of flurbiprofen or of a pharmaceutically acceptable salt of flurbiprofen.

Pharmaceutical formulations according to the present invention may also comprise one or more pharmaceutically acceptable excipient(s). Such pharmaceutically acceptable excipients include, but are not limited to disintegrants, fillers, glidants, lubricants, coating agents, surface active agents, etc. and any mixtures thereof.

Suitable disintegrants include, but are not limited to alginic acid and alginates, ion-exchange resins, magnesium aluminum silicate, sodium dodecyl sulfate, sodium carboxymethyl cellulose, croscarmellose sodium, cross-linked PVP, carboxymethyl cellulose calcium, docusate sodium, guar gum, low-substituted HPC, polacrilin potassium, poloxamer, povidone, sodium alginate, sodium glycine carbonate and sodium lauryl sulfate, etc., and any mixtures thereof.

Suitable fillers include sugars, mannitol, sorbitol, sucrose, inorganic salts, calcium salts, polysaccharides, dextrose, dicalcium phosphate, sodium chloride, dextrates, lactitol, maltodextrin, sucrose-maltodextrin mixtures, xylitol, trehalose, heavy magnesium carbonate, galen IQ (isomalt), LudiFlash (mannitol, crospovidone and polyvinyl acetate), Pharmaburst, Panexcea (microcrystalline cellulose, HPMC and crospovidone), F-Melt.

Suitable binders include, but are not limited to polyvinylpyrrolidone, polyethylene glycol, polyvinyl alcohol, hydroxypropyl methyl cellulose, carboxymethyl cellulose, methyl cellulose and other cellulose derivatives, starch, collagen, gelatin, sugars, aluminum hydroxide, polyvinyl alcohol, carrageenan, bentonite, laponite, and other inorganic agents; cetostearyl alcohol, polyoxyethylene-alkyl ethers, and other surfactants, starch mucilage, acacia mucilage, polydextrose, polyethylene oxide, pullulan, guar gum, xanthan gum and similar agents, and any mixtures thereof.

## Claims

1. An orally-disintegrating pharmaceutical formulation, **characterized by** comprising flurbiprofen and polyvinylcaprolactam-polyvinyl acetate-polyethylene glycol graft copolymer.

2. The orally-disintegrating pharmaceutical formulation according to Claim 1, wherein said formulation is obtained by means of a hot-melt method not involving any liquid solvent during the granulation phase.

3. The pharmaceutical formulation according to any of the preceding claims, wherein the proportion range of flurbiprofen to polyvinylcaprolactam-polyvinyl acetate-polyethylene glycol graft copolymer is 0.1 to 10, preferably 0.15 to 5, and more preferably 0.2 to 2.

4. The pharmaceutical formulation according to any of the preceding claims, further comprising at least one or more excipient(s).

5. The pharmaceutical formulation according to any of the preceding claims, wherein one or more pharmaceutically acceptable excipients are selected from the group consisting of disintegrants, diluents, plasticizers, binders, glidants, lubricants, sweeteners, flavoring agents, and coloring agents.

6. The pharmaceutical formulation according to any of the preceding claims, wherein said disintegrants are selected from the group consisting of at least one or a mixture of croscarmellose sodium, crospovidone, low-substituted hydroxypropyl cellulose, sodium starch glycolate, and soy polysaccharides.

7. The pharmaceutical formulation according to any of the preceding claims, wherein the proportion by weight of flurbiprofen to disintegrants is in the range of 1:10 to 10:1.

8. The pharmaceutical formulation according to any of the preceding claims, wherein the disintegrant is preferably croscarmellose sodium.

9. The pharmaceutical formulation according to any of the preceding claims, wherein said diluents are preferably mannitol and/or microcrystalline cellulose.

10. The pharmaceutical formulation according to any of the preceding claims, comprising at least one or a mixture of castor oil, glycerin, citrate esters (acetyl tri-n-butyl citrate, acetyl triethyl citrate, tri-n-butyl citrate, triethyl citrate), sebacate esters (dibutyl sebacate), phthalate esters (diethyl phthalate, dibutyl phthalate, dioctyl phthalate), mineral oils, polyethylene oxide, triacetine, diethyl phthalate, fatty acid esters (butyl stearate, glycerol monostearate, stearyl alcohol), polyethylene glycol, propylene glycol.

11. The pharmaceutical formulation according to any of the preceding claims, wherein said binders are preferably polyvinylpyrrolidone and/or hydroxypropyl methyl cellulose.

12. The pharmaceutical formulation according to any of the preceding claims, wherein said lubricants are preferably magnesium stearate and sodium stearyl fumarate.

13. The pharmaceutical formulation according to any of the preceding claims, wherein said glidant is preferably colloidal silicon dioxide.

14. The pharmaceutical formulation according to any of the preceding claims, wherein said sweeteners are preferably aspartam, sucralose and/or saccharine.

15. The pharmaceutical formulation according to any of the preceding claims, wherein said flavoring agents are preferably menthol and/or fruit extracts.

16. The pharmaceutical formulation according to any of the preceding claims, consisting of
(a) approximately 5 to 60% by weight of flurbiprofen or a pharmaceutically acceptable salt of flurbiprofen;
(b) approximately 0.25 to 20% by weight of polyvinylcaprolactam-polyvinyl acetate-polyethylene glycol graft copolymer,
(c) approximately 0.1 to 30% by weight of croscarmellose sodium,
(d) approximately 1 to 60% by weight of mannitol,
(e) approximately 1 to 30% by weight of microcrystalline cellulose,
(f) approximately 1 to 20% by weight of polyvinylpyrrolidone,
(g) approximately 0.01 to 5% by weight of colloidal silicon dioxide,
(h) approximately 0.1 to 5% by weight of magnesium stearate,
(i) approximately 0.01 to 5% by weight of sucralose,
(j) approximately 0.01 to 5% by weight of menthol and/or fruit extracts.

## Patentansprüche

1. Im Mund zerfallende pharmazeutische Formulierung, **dadurch gekennzeichnet, dass** sie Flurbiprofen und Polyvinylcaprolactam-Polyvinylacetat-Polyethylenglycol-PfropfCopolymer umfasst.

2. Im Mund zerfallende pharmazeutische Formulierung nach Anspruch 1, wobei die Formulierung mittels eines Heißschmelzverfahrens erhalten wird, das während der Granulierungsphase kein flüssiges Lösungsmittel beinhaltet.

3. Pharmazeutische Formulierung nach einem der vorangehenden Ansprüche, wobei der Verhältnisbereich von Flurbiprofen zu Polyvinylcaprolactam-Polyvinylacetat-Polyethylenglycol-Pfropf-Copolymer 0,1 bis 10, bevorzugt 0,15 bis 5 und bevorzugter 0,2 bis 2 ist.

4. Pharmazeutische Formulierung nach einem der vorangehenden Ansprüche, weiterhin umfassend wenigstens ein oder mehrere Bindemittel.

5. Pharmazeutische Formulierung nach einem der vorangehenden Ansprüche, wobei das eine oder die mehreren pharmazeutisch akzeptablen Bindemittel ausgewählt sind aus der Gruppe, bestehend aus Zersetzungsmitteln. Verdünnungsmitteln, Weichmachern, Bindemitteln, Gleitstoffen, Schmiermitteln, Süßstoffen, Aromastoffen und Farbstoffen.

6. Pharmazeutische Formulierung nach einem der vorangehenden Ansprüche, wobei die Zersetzungsmittel ausgewählt sind aus der Gruppe, bestehend aus wenigstens einem oder einer Mischung von Croscarmellose-Natrium, Crospovidone, gering substituierter Hydroxypropylcellulose, Natriumstärkeglycolat und Soja-Polysacchariden.

7. Pharmazeutische Formulierung nach einem der vorangehenden Ansprüche, wobei das Gewichtsverhältnis von Flurbiprofen zu Zersetzungsmitteln im Bereich von 1:10 bis 10:1 ist.

8. Pharmazeutische Formulierung nach einem der vorangehenden Ansprüche, wobei das Zersetzungsmittel bevorzugt Croscarmellose-Natrium ist.

9. Pharmazeutische Formulierung nach einem der vorangehenden Ansprüche, wobei die Verdünnungsmittel bevorzugt Mannitol und/oder mikrokristalline Cellulose sind.

10. Pharmazeutische Formulierung nach einem der vorangehenden Ansprüche, umfassend wenigstens eines oder eine Mischung von Rizinusöl, Glycerin, Citratester (Acetyl-tri-n-butylcitrat, Acetyl-triethylcitrat, Tri-n-butylcitrat, Triethylcitrat), Sebacatester (Dibutylsebacat), Phthalatester (Diethylphthalat, Dibutylphthalat, Dioctylphthalat), Mineralöle, Polyethylenoxid, Triacetin, Diethylphthalat, Fettsäureester (Butylstearat, Glycerolmonostearat, Stearylalkohol), Polyethylenglycol, Propylenglycol.

11. Pharmazeutische Formulierung nach einem der vorangehenden Ansprüche, wobei die Bindemittel bevorzugt Polyvinylpyrrolidon und/oder Hydroxypropylmethylcellulose sind.

12. Pharmazeutische Formulierung nach einem der vorangehenden Ansprüche, wobei die Schmiermittel bevorzugt Magnesiumstearat und Natriumstearylfumarat sind.

13. Pharmazeutische Formulierung nach einem der vorangehenden Ansprüche, wobei das Gleitmittel bevorzugt kolloidales Siliciumdioxid ist.

14. Pharmazeutische Formulierung nach einem der vorangehenden Ansprüche, wobei die Süßstoffe bevorzugt Aspartam, Sucralose und/oder Saccharin sind.

15. Pharmazeutische Formulierung nach einem der vorangehenden Ansprüche, wobei die Aromastoffe bevorzugt Menthol und/oder Fruchtextrakte sind.

16. Pharmazeutische Formulierung nach einem der vorangehenden Ansprüche, bestehend aus
(a) näherungsweise 5 bis 60 Gew.% Flurbiprofen oder eines pharmazeutisch akzeptablen Salzes von Flurbiprofen;
(b) näherungsweise 0,25 bis 20 Gew.% von Polyvinylcaprolactam-Polyvinylacetat-Polyethylenglycol-Pfropf-Copolymer,
(c) näherungsweise 0,1 bis 30 Gew.% Croscarmellose-Natrium,
(d) näherungsweise 1 bis 60 Gew.% Mannitol,
(e) näherungsweise 1 bis 30 Gew.% mikrokristalliner Cellulose,
(f) näherungsweise 1 bis 20 Gew.% Polyvinylpyrrolidon,
(g) näherungsweise 0,01 bis 5 Gew.% kolloidales Siliciumdioxid,
(h) näherungsweise 0,1 bis 5 Gew.% Magnesiumstearat,
(i) näherungsweise 0,01 bis 5 Gew.% Sucralose,
(j) näherungsweise 0,01 bis 5 Gew.% Menthol und/oder Fruchtextrakte.

## Revendications

1. Formulation pharmaceutique se désintégrant par voie orale, **caractérisée en ce qu'**elle comprend du flurbiprofène et un copolymère greffé de polyvinylecaprolactame-polyvinyle acétate de polyéthylène-glycol.

2. Formulation pharmaceutique se désintégrant par voie orale suivant la revendication 1, ladite formulation étant obtenue au moyen d'un procédé de fusion à chaud n'impliquant aucun solvant liquide durant la phase de granulation.

3. Formulation pharmaceutique suivant une quelconque des revendications précédentes, dans laquelle le rapport en poids de flurbiprofène au copolymère greffé de polyvinylecaprolactame-polyvinyle acétate de polyéthylène-glycol s'élève à une valeur de 0,1 : 10, de préférence de 0,15 : 5 et plus préférentiellement de 0,2 : 2.

4. Formulation pharmaceutique suivant une quelconque des revendications précédentes comprenant, en plus, au moins un ou plusieurs excipients.

5. Formulation pharmaceutique suivant une quelconque des revendications précédentes, dans laquelle un ou plusieurs excipients pharmaceutiquement acceptables sont sélectionnés parmi le groupe consistant en désintegrants, diluants, plastifiants, liants, agents de glissement, lubrifiants, édulcorants, agents aromatiques, agents de coloration.

6. Formulation pharmaceutique suivant une quelconque des revendications précédentes, dans laquelle lesdits désintégrants sont sélectionnés parmi le groupe consistant en au moins une parmi les substances suivantes: sodium de croscarmellose, crospovidone, hydroxyprolyl-cellulose faiblement substituée, glycolate d'amidon sodique et polysaccharides de soja ou en un mélange de celles-ci.

7. Formulation pharmaceutique suivant une quelconque des revendications précédentes, dans laquelle le rapport en poids de flurbiprofène aux désintégrants s'élève à une valeur de 1 :10 à 10 :1.

8. Formulation pharmaceutique suivant une quelconque des revendications précédentes, dans laquelle le désintégrant est de préférence du sodium de croscarmellose.

9. Formulation pharmaceutique suivant une quelconque des revendications précédentes, dans laquelle lesdits diluants sont de préférence du mannitol et/ou de la cellulose microcristalline.

10. Formulation pharmaceutique suivant une quelconque des revendications précédentes, comprenant au moins une parmi les substances suivantes: huile de ricin, glycérine, esters citrates (acétyle tri-n-butyle citrate, acétyle triéthyle citrate, tri-n-butyle citrate, triéthyle citrate), esters sebaçates (sébaçate de dibutyle) esters phtalates (phtalate de diéthyle, phtalate de dibutyle, phtalate de dioctyle), huiles minérales, oxyde de polyéthylène, triacétine, phtalate de diéthyle, esters d'acides gras, (stéarate de butyle, monostéarate de glycérine, alcool stéarylique), polyéthylène-glycol, polypropylène glycol ou un mélange de celles-ci.

11. Formulation pharmaceutique suivant une quelconque des revendications précédentes, dans laquelle lesdits liants sont de préférence du polyvinylpyrrolidone et/ou de la hydroxypropylméthylcellulose.

12. Formulation pharmaceutique suivant une quelconque des revendications précédentes, dans laquelle lesdits lubrifiants sont de préférence du stéarate de magnésium, du stéarylfumarate de sodium.

13. Formulation pharmaceutique suivant une quelconque des revendications précédentes, dans laquelle l'agent de glissement est de préférence du dioxyde de silicium colloïdal.

14. Formulation pharmaceutique suivant une quelconque des revendications précédentes, dans laquelle les agents édulcorants sont de préférence de l'aspartame, du sucralose et/ou de la saccharine.

15. Formulation pharmaceutique suivant une quelconque des revendications précédentes, dans laquelle les agents aromatiques sont de préférence du menthol et/ou des extraits de fruits.

16. Formulation pharmaceutique suivant une quelconque des revendications précédentes, consistant en
(a) approximativement 5 à 60% en poids de flurbiprofène ou d'un sel pharmaceutiquement acceptable de flurbiprofène,
(b) approximativement 0,25 à 20% en poids de copolymère greffé de polyvinylecaprolactame-polyvinyle acétate de polyéthylène-glycol,
(c) approximativement 0,1 à 30% en poids de sodium de croscarmellose,
(d) approximativement 1 à 60% en poids de mannitol,
(e) approximativement 1 à 30% en poids de cellulose microcristalline,
(f) approximativement 1 à 20% en poids de polyvinylpyrrolidone,
(g) approximativement 0,01 à 5% en poids de dioxyde de silicium colloïdal,
(h) approximativement 0,1 à 5% en poids de stéarate de mangésium,
(i) approximativement 0,01 à 5% en poids de sucralose,
(j) approximativement 0,01 à 5% en poids de menthol et/ou d'extraits de fruits.
